(19) Europäisches Patentamt
European Patent Office
Office européen des brevets

(11) Numéro de publication: **0 211 728 B1**

(12) **FASCICULE DE BREVET EUROPEEN**

(45) Date de publication du fascicule du brevet: **31.01.90**

(51) Int. Cl.⁴: **C 07 D 457/04**

(21) Numéro de dépôt: **86401540.9**

(22) Date de dépôt: **10.07.86**

(54) **Procédé de préparation des dérivés N-méthyles du dihydrolysergate de méthyle ou du méthoxylumilysergate de méthyle.**

(30) Priorité: **11.07.85 FR 8510622**

(43) Date de publication de la demande: **25.02.87 Bulletin 87/9**

(45) Mention de la délivrance du brevet: **31.01.90 Bulletin 90/5**

(84) Etats contractants désignés: **AT BE CH DE FR GB IT LI LU NL SE**

(56) Documents cités:
**EP-A- 0 000 533**

**Helv. Chim. Acta 40, 1721 (1957)**

**Le dossier contient des informations techniques présentées postérieurement au dépôt de la demande et ne figurant pas dans le présent fascicule.**

(73) Titulaire: **RHONE-POULENC SANTE, 20, avenue Raymond Aron, F-92160 Antony (FR)**

(72) Inventeur: **Gervais, Christian, 6 allée Marcel Achard, F-69100 Villeurbanne (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne un nouveau procédé de préparation des dérivés N-méthylés du dihydrolysergate de méthyle et du méthoxylumilysergate de méthyle de formule générale:

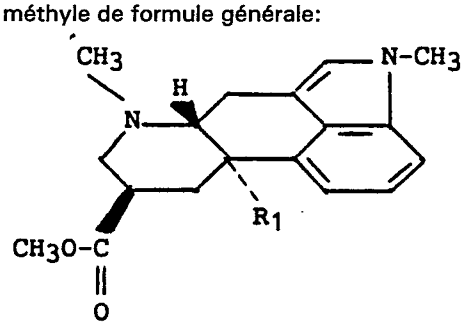

(I)

dans laquelle $R_1$ représente un atome d'hydrogène ou un radical méthoxy.

Les produits de formule générale (I) sont des intermédiaires particulièrement intéressants dans la synthèse de produit à activité pharmacologique qui sont décrits dans le brevet américain US 3 228 943.

L'art antérieur enseigne que la méthylation de l'atome d'azote indolique des dérivés de l'ergoline doit être réalisée au moyen d'un agent de méthylation tel qu'un halogénure de méthyle ou le sulfate de méthyle en présence d'une base telle que l'amidure de potassium, dans l'ammoniac liquide selon F. Troxler, Helv. Chim. Acta, 40, 1721 (1957) ou l'amidure de sodium dans l'ammoniac liquide selon le brevet suisse CH 386 441 ou le brevet américain US 3 113 133. Ces procédés nécessitent la mise en œuvre de sodium ou de potassium et le maintien d'une température voisine de −40°C ce qui, sur le plan industriel, implique des investissements importants. Par ailleurs, ces procédés peuvent conduire à des produits secondaires de méthylation.

Dans la demande de brevet européen publiée sous le numéro 533 est décrite la méthylation du lumilysergate de méthyle en présence de soude à 45% utilisée en catalyse par transfert de phase liquide-liquide. Une telle réaction doit être soigneusement contrôlée pour éviter la saponification de la fonction ester méthylique.

Dans la demande de brevet européen publiée sous le numéro 4664 est décrite la méthylation du méthoxy-10α lumilysergol en présence de potasse solide dans le diméthylsulfoxyde, mais une telle réaction n'est pas sélective et il se forme une proportion importante d'éther méthylique sur la fonction alcool primaire.

Dans le cas de l'indole non substitué, il est connu d'effectuer la méthylation en présence d'hydrure de sodium dans l'hexaméthylphosphotriamide [cf. G.H. Rubottom et J.C. Chabala, Synthesis, 566 (1973)] ou l'amidure de sodium, l'amidure de lithium ou le bromure d'éthylmagnésium dans le tétrahydrofuranne, l'éther, le benzène ou l'hexaméthylphosphotriamide [M.G. Reinecke et coll., J. Org. Chem., 37 (20) 3066 (1977)].

Il a maintenant été trouvé, et c'est ce qui fait l'objet de la présente invention, que les produits de formule générale (I) peuvent être obtenus à partir des produits de formule générale:

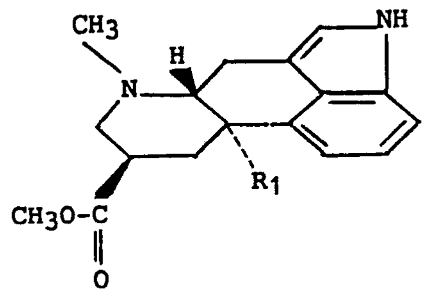

(II)

dans laquelle $R_1$ est défini comme précédemment par action d'un agent de méthylation en présence d'un sel d'ammonium quaternaire et en présence d'un alcoolate métallique solide de formule générale:

$$CH_3\text{-}OM \qquad (III)$$

dans laquelle M représente un atome de métal alcalin tel qu'un atome de sodium, et d'un agent déshydratant choisi parmi les esters de formule générale:

$$R_2\text{-}COO\ CH_3 \qquad (IV)$$

dans laquelle $R_2$ représente un radical alcoyle, alcoyloxy, alcoyloxycarbonyle ou aryle.

Le procédé selon l'invention est caractérisé par l'emploi d'un agent de méthylation en présence d'un catalyseur de transfert de phase liquide/solide associé à un agent déshydratant choisi parmi les esters de formule générale (IV) et d'une base non transestérifiante.

La mise en œuvre du procédé est généralement réalisée à une température comprise entre −10 et 80°C et elle ne fait pas appel à une tecnologie particulière.

Par ailleurs, l'emploi d'un ester de formule générale (IV) permet de contrôler la réaction parasite de saponification même en présence de traces d'eau contenues dans les réactifs.

Généralement, le procédé selon l'invention est mis en œuvre dans un solvant organique aprotique choisi parmi les éthers tels que le tétrahydrofuranne, les esters tels que l'acétate de méthyle, le carbonate de méthyle ou le benzoate de méthyle ou leur mélange.

Les agents déshydratants peuvent être choisis parmi les esters tels que l'acétate de méthyle, le benzoate de méthyle ou le carbonate de méthyle, éventuellement en présence d'un agent co-déshydratant choisi parmi le magnésium ou le diméthoxymagnésium. Les agents déshydratants peuvent être utilisés comme solvants.

Le catalyseur de transfert de phase liquide/solide est généralement choisi parmi les sels d'ammonium quaternaires tels que les halogénures, sulfate de tétraalcoylammonium comme, par exemple, le chlorure de tétrabutylammonium.

Les bases sont généralement choisies parmi les alcoolates de métaux alcalins comme le méthylate de sodium.

L'agent de méthylation est, de préférence, le sulfate de méthyle.

Par rapport au produit à méthyler, on utilise généralement un excès de base dans un rapport molaire

compris entre 2 et 10, un excès d'ester de formule générale (III) dans un rapport molaire compris entre 1 et 500 et le catalyseur de transfert de phase dans un rapport molaire compris entre 0,05 et 2. Il est particulièrement avantageux d'utiliser un léger excès d'agent de méthylation.

Les produits de formule générale (I) sont isolés du mélange réactionnel selon les techniques habituelles.

Les exemples suivants montrent comment l'invention peut être mise en pratique.

*Exemple 1*

Dans un réacteur, maintenu à l'abri de l'humidité, on introduit 1,758 g de méthoxy-10α lumilysergate de méthyle (5,6 m.moles), 115 $cm^3$ de tétrahydrofuranne anhydre, 15 $cm^3$ de benzoate de méthyle (120,5 m.moles) et 1,574 g de chlorure de tétrabutylammonium (5,6 m.moles). Après dissolution complète, on introduit, sous agitation, 2,5 g de méthylate de sodium en poudre (46,3 m.moles). Après 5 minutes d'agitation, on ajoute en 5 heures une solution de 0,776 g de sulfate de diméthyle (6,16 m.moles) dans du tétrahydrofuranne anhydre.

Le mélange réactionnel est versé dans 86 $cm^3$ d'acide sulfurique 0,65N maintenu à 0°C. La phase aqueuse (pH = 2) est lavée par 35 $cm^3$ d'acétate d'éthyle, est alcalinisée jusqu'à pH = 9 par addition d'ammoniaque puis est extraite par 4 fois 70 $cm^3$ d'acétate d'éthyle. Les phases organiques réunies sont séchées. Après évaporation du solvant, on obtient, avec un rendement de 80-85%, le méthoxy-10α méthyl-1 lumilysergate de méthyle dont le titre, déterminé par chromatographie liquide à haute performance et par acidimétrie, est supérieur à 99%.

*Example 2*

On opère comme dans l'exemple 1 mais en utilisant:

- 0,314 g de méthoxy-10α lumilysergate de méthyle (1 m.mole)
- 51,5 g de benzoate de méthyle (378,6 m.moles)
- 0,277 g e chlorure de tétrabutylammonium (1 m.mole)
- 0,432 g de méthylate de sodium (8 m.moles)
- 0,315 g de sulfate de diméthyle (2,5 m.moles)

L'addition de sulfate de diméthyle est effectuée après avoir agité le mélange réactionnel pendant 17 heures à une température voisine de 20°C.

Le taux de transformation du méthoxy-10α lumilysergate de méthyle est de 92%.

Le rendement en méthoxy-10α méthyl-1 lumilysergate de méthyle est de 81%.

*Exemple 3*

On opère comme dans l'exemple 1 mais en utilisant:

- 0,314 g de méthoxy-10α lumilysergate de méthyle (1 m.mole)
- 11,1 $cm^3$ d'un mélange tétrahydrofuranne-acétate de méthyle (1-1 en volumes) comme solvant
- 0,277 g de chlorure de tétrabutylammonium (1 m.mole)
- 7,4 g d'acétate de méthyle (0,1 mole)
- 0,432 g de méthylate de sodium (8 m.moles)
- 0,441 g de sulfate de diméthyle (0,0035 m.mole).

L'addition du sulfate de méthyle est effectuée après avoir agité le mélange réactionnel pendant 2 heures 20 minutes à une température voisine de 20°C.

Le taux de transformation du méthoxy-10α lumilysergate de méthyle est de 92%.

Le rendement en méthoxy-10α méthyl-1 lumilysergate de méthyle est de 80%.

*Exemple 4*

On opère comme dans l'exemple 1 mais en utilisant:

- 0,314 g de méthoxy-10α lumilysergate de méthyle (1 m.mole)
- 22,2 $cm^3$ d'un mélange tétrahydrofuranne-carbonate de méthyle (1-1 en volumes) comme solvant
- 0,277 g de chlorure de tétrabutylammonium (1 m.mole)
- 4,5 g de carbonate de méthyle (50 m.moles)
- 0,11 g de diméthoxymagnésium (2 m.moles)
- 0,432 g de méthylate de sodium (8 m.moles)
- 0,167 g de sulfate de diméthyle (1,33 m.moles).

L'addition de sulfate de diméthyle est effectuée après avoir agité le mélange réactionnel pendant 2 heures à une température voisine de 20°C.

Le taux de transformation du méthoxy-10α lumilysergate de méthyle est de 93%.

Le rendement en méthoxy-10α méthyl-1 lumilysergate de méthyle est de 82%.

**Revendications**

1. Procédé de préparation des dérivés N-méthylés du dihydrolysergate de méthyle et du méthoxylumilysergate de méthyle de formule générale:

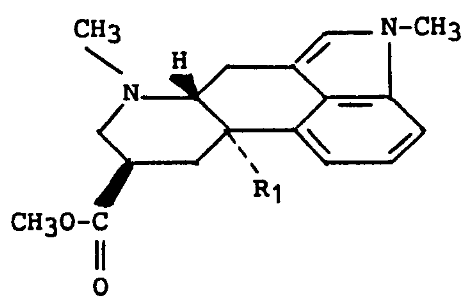

dans laquelle $R_1$ représente un atome d'hydrogène ou un radical méthoxy caractérisé en ce que, sur un produit de formule générale:

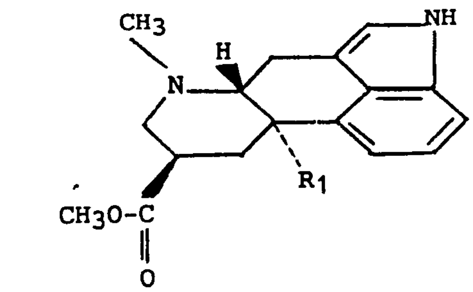

dans laquelle $R_1$ est défini comme précédemment, on fait réagir un agent de méthylation en présence d'un sel d'ammonium quaternaire et en présence de méthylate de sodium et d'un agent déshydratant choisi parmi les esters de formule générale:

$$R_2\text{-COO } CH_3$$

dans laquelle $R_2$ représente un radical alcoyle, alcoyloxy, alcoyloxycarbonyle ou aryle en utilisant un excès de base dans un rapport molaire compris entre 2 et 10, un excès d'ester dans un rapport molaire compris entre 1 et 500 et le catalyseur de transfert de phase dans un rapport molaire compris entre 0,05 et 2.

2. Procédé selon la revendication 1, caractérisé en ce que l'on opère dans un solvant aprotique choisi parmi le tétrahydrofuranne et les esters et leurs mélanges.

3. Procédé selon la revendication 2, caractérisé en ce que les esters sont choisis parmi l'acétate de méthyle, le benzoate de méthyle ou le carbonate de méthyle.

4. Procédé selon la revendication 1, caractérisé en ce que l'agent de méthylation est le sulfate de méthyle.

**Patentansprüche**

1. Verfahren zur Herstellung von N-Methylderivaten von Methyldihydrolysergat und Methylmethoxylumilysergat der allgemeinen Formel:

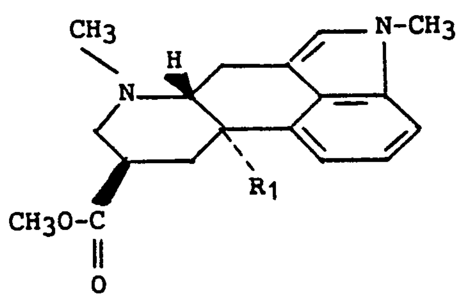

n welcher $R_1$ ein Wasserstoffatom oder einen Methoxyrest darstellt, dadurch gekennzeichnet, daß man auf eine Verbindung der allgemeinen Formel:

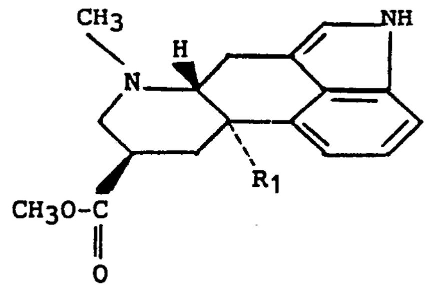

in welcher $R_1$ die obige Bedeutung hat, ein Methylierungsmittel in Gegenwart eines quaternären Ammoniumsalzes und in Gegenwart von Natriummethylat und einem Entwässerungsmittel, ausgewählt aus den Estern der allgemeinen Formel:

$$R_2\text{-COO } CH_3$$

in welcher $R_2$ einen Alkyl-, Alkyloxy-, Alkyloxycarbonyl- oder arylrest darstellt, einwirken läßt, indem man einen Überschuss an Base in einem molaren Verhältnis zwischen 2 und 10, einen Überschuß an Ester in einem molaren Verhältnis zwischen 1 und 500 und den Phasenübertragungskatalysator in einem molaren Verhältnis zwischen 0,05 und 2 einsetzt.

2. Verfahren nach Anspruch 1, dadurch gekennzeichnet, daß man in einem aprotischen Lösungmittel, ausgewählt aus Tetrahydrofuran und den Estern und ihren Mischungen, arbeitet.

3. Verfahren nach Anspruch 2, dadurch gekennzeichnet, daß die Ester ausgewählt werden aus Methylacetat, Methylbenzoat oder Methylcarbonat.

4. Verfahren nach Anspruch 1, dadurch gekennzeichnet, daß das Methylierungsmittel Methylsulfat ist.

**Claims**

1. A process for preparing N-methyl derivatives of methyl dihydrolysergate and methyl methoxylumilysergate, of general formula:

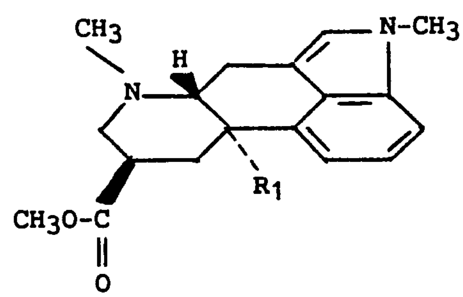

in which $R_1$ denotes a hydrogen atom or a methoxy radical, wherein a methylating agent is reacted with a product of general formula:

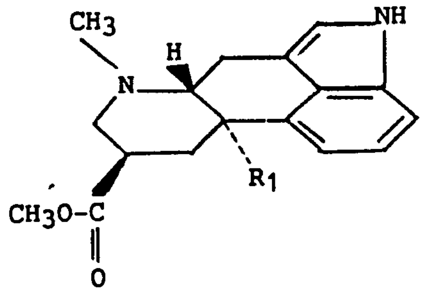

in which $R_1$ is defined as above, in the presence of a quaternary ammonium salt and in the presence of sodium methylate and a dehydrating agent chosen from the esters of general formula:

$$R_2\text{-COO } CH_3$$

in which $R_2$ denotes an alkyl, alkyloxy, alkyloxycarbonyl or aryl radical, using an excess of base in a mole ratio of between 2 and 10, an excess of ester in a mole ratio of between 1 and 500 and the phase transfer catalyst in a mole ratio of between 0.05 and 2.

2. The process according to claim 1, wherein the procedure is performed in an aprotic solvent chosen from tetrahydrofuran and esters and mixtures thereof.

3. The process according to claim 2, wherein the esters are chosen from methyl acetate, methyl benzoate or methyl carbonate.

4. The process according to claim 1, wherein the methylating agent is methyl sulphate.